# EUROPEAN PATENT SPECIFICATION

(11) **EP 3 503 884 B1**
(45) Date of publication and mention of the grant of the patent: **21.07.2021**
(21) Application number: 17745509.4
(22) Date of filing: 30.06.2017
(51) Int. Cl.: A61K 31/351, A61K 31/4045, A61P 17/04, A61K 31/404, A61K 45/06, A61K 9/00, A61K 9/06, A61K 47/22, A61K 47/24, A61K 47/26, A61K 47/36, A61K 47/38, A61K 47/44, A61K 47/46

(54) **PHARMACEUTICAL COMPOSITION FOR USE IN TREATING PRURITUS AND/OR ITCH**
PHARMAZEUTISCHE ZUSAMMENSETZUNG ZUR VERWENDUNG BEI DER BEHANDLUNG VON PRURITUS UND/ODER JUCKREIZ
COMPOSITION PHARMACEUTIQUE UTILISÉE POUR LE TRAITEMENT DU PRURIT ET/OU DES DÉMANGEAISONS

(30) Priority: 23.08.2016 US 201662378435 P
(43) Date of publication of application: 03.07.2019
(73) Proprietor: Neurim Pharmaceuticals Ltd., 6971039 Tel Aviv (IL)
(72) Inventor: LAUDON, Moshe, 4444435 Kfar Saba (IL)
(74) Representative: PGA S.p.A., Milano, Succursale di Lugano
(86) International application number: PCT/IB2017/053989
(87) International publication number: WO 2018/037295

(56) References cited:
- EP-A1- 0 834 317
- WO-A2-2007/093880
- WO-A2-2014/144545
- US-A1- 2013 324 583

## Description

### FIELD OF THE INVENTION

The present disclosure relates generally to treating pruritus and/or itch, to the use of pyrone-indole compounds in the manufacture of a medicament useful for treating pruritus and/or itch, and to a medicament for use in alleviating itch or pruritus in a patient suffering from dermatological and non-dermatological conditions leading to such symptoms.

### BACKGROUND OF THE INVENTION

Itch is an uncomfortable sensation on the skin that causes a desire to scratch (Bautista, Wilson et al. 2014). Pruritus, defined as a sensation driving the urge to scratch, may be acute (<6 weeks) or chronic (>6 weeks) (Lavery, Stull et al. 2016). This symptom can significantly impair the quality of life and sleep of affected patients. Moreover the cumulative effect of such disruptions may influence mortality. The dialysis outcomes and practice patterns (DOPPS) study showed a 17% increased mortality risk among pruritic hemodialysis patients, which was attributed in part to decreased quality of sleep. Pruritus is severe itching of the skin, as a symptom of various ailments. Itch can be classified by etiology (Chuquilin, Alghalith et al. 2016). Pruriceptive itch originates from the activation of primary afferent nerve terminals (e.g., insect bites). Neurogenic itch is caused by central nervous system injury or activation without the activation of sensory nerve terminals (e.g., renal disease and kidney failure). Psychogenic itch results from a pure central psychic processing disorder in the absence of skin pathology or underlying medical disease.

**A** myriad of both dermatological and non-dermatological conditions may cause itch and/or pruritus. In clinical practice, patients with chronic pruritus from any cause commonly complain of nocturnal involvement. Nocturnal pruritus is common in patients with chronic itch and negatively affects sleep quality. Lack of sleep can have both immediate and long-term effects that lead to medical, social and financial ramifications. Physicians must be aware of the numerous etiologies of itch and nocturnal pruritus and deliver treatment with a patient-centered approach. Aptly tailored management can help alleviate distress and reduce the complications that result from repetitive scratching.

**Table 1 depicts some of the more common etiologies of pruritus.**

| Category | Disease |
|---|---|
| Dermatological | Atopic dermatitis |
| | Psoriasis |
| | Chronic idiopathic urticaria |
| | Infestations (scabies, bed bugs, pediculosis, pinworms) |
| | Lichen planus |
| | Lichen simplex chronicus |
| | Prurigo nodularis |
| | Advanced age (senile) pruritus |
| | Brachioradial pruritus |
| Non-Dermatological | Liver disease |
| | Chronic kidney disease |
| | Hematopoietic disorders |
| | Neurological (e.g., brachioradial pruritus) |
| | Psychological (delusional ideations, depression, |
| | schizophrenia, stress) |
| | Substance abuse |
| | Advanced age (senile) pruritus |
| | Restless legs syndrome |

Many patients suffer from nocturnal pruritus, which can decrease quality of life and affect mortality in hemodialysis patients (Lavery, Stull et al. 2016). Nocturnal pruritus may occur in all sleep stages but is most prevalent in stages N1 and N2. Conditions with a high prevalence of nocturnal pruritus include advanced age (senile) pruritus, atopic dermatitis, prurigo nodularis, psoriasis, brachioradial pruritus and chronic idiopathic urticaria. Two major dermatological conditions that cause nocturnal pruritus are atopic dermatitis and psoriasis. Atopic dermatitis is a chronic condition with a prevalence of 7.2% in adults and 10.7% in children in the USA. Chronic pruritus is one of the most common presenting complaints in atopic dermatitis patients with a point prevalence ranging from 87%-100% (Lauffer and Ring 2016). Clinically, atopic dermatitis manifests as pruritic erythematous lesions with associated excoriations, lichenification and/or superimposed infection. Psoriasis is a chronic inflammatory immune-mediated skin disorder. Prevalence varies greatly by country with a prevalence of 0.91% in the USA and 8.5% in Norway. Activation of the immune system triggers cytokine cascades involved in the different psoriatic cutaneous manifestations. Chronic idiopathic urticaria (also called chronic spontaneous urticaria) is defined as itchy hives that last for at least 6 weeks, with or without angioedema, and that have no apparent external trigger. The condition generally has a prolonged duration of 1 to 5 years (persisting for >5 years in 11 to 14% of patients) and has a detrimental effect on patients' emotional and physical health-related quality of life. The impairment accompanying this disorder has been likened to that seen in patients with ischemic heart disease, with patients feeling a similar lack of energy, social isolation, and emotional upset as those with heart disease. (Maurer, Rosen et al. 2013).

Itch neurons (pruriceptors) are polymodal and respond to stimulus other than pruritogens, including heat and capsaicin (Chuquilin, Alghalith et al. 2016). Although pruriceptive neurons are a subset of nociceptive C-fiber neurons in DRG, recent progress indicates that there are separate labeled lines for itch and pain in the spinal cord. The selectivity theory of itch is the most largely accepted theory to explain why a stimulus is able to cause itch and not pain. Pruriceptors are a subset of nociceptors, and this theory postulates that itch occurs when these selective itch neurons are activated alone, while the sensation of pain dominates when itch and pain neurons are activated together. In this manner, inhibition of the itch pathway occurs via the nociceptive only neurons.

Numerous itch mediators (pruritogens) and receptors (pruriceptors) have been identified, of which the best understood are histamine, proteases, opioids, substance P, the Mas-related G protein-coupled receptor (Mrgpr) family, and calcitonin gene-related peptide (CGRP). Circadian rhythms may play a role in nocturnal pruritus. The hypothalamus-pituitary-adrenal axis is a complex system that releases corticosteroids through a continuous negative feedback mechanism.

H1 -antihistamines are the current mainstay for initial treatment and are the only agents approved for use in patients with chronic idiopathic urticarial and nocturnal pruritus (Furue and Kadono 2015). However, a majority of patients do not have a response to H1-antihistamines, even when the drugs are administered at three to four times their approved doses. Treatment options for patients who do not have a response to H1-antihistamines include the use of H2-antihistamines, leukotriene-receptor antagonists, systemic glucocorticoids, cyclosporine, hydroxychloroquine, dapsone, methotrexate, sulfasalazine, and intravenous immune globulin. Mirtazapine, an atypical antidepressant that antagonizes noradrenergic (α1, α2), serotonergic (5-HT2, 5-HT3), and histaminergic (H1) receptors, has demonstrated efficacy in relieving nocturnal itch of varied etiology. None of these agents has yet received regulatory approval for the treatment of itch. In addition, the data supporting the use of these drugs are limited, and long-term use of some of the agents can be associated with substantial side effects.

Transient receptor potential vanilloid type 1 (TRPV1) is a non-selective cation channel widely expressed in skin tissues, and peripheral sensory nerve fibers. Activation of TRPV1 releases neuropeptides; the resulting neurogenic inflammation is believed to contribute to the development of pruritus. However, in a recent study topical administration of a TRPV1 antagonist failed to be of symptomatic benefit for histaminergic or non-histaminergic induced itch in humans (Gibson, Robertson et al. 2014).

Smooth muscle cells of cutaneous arterioles and arteriole-venule shunts (AVS) in the skin express sodium channel Nav1.7. Moreover, Nav1.7 is expressed by endothelial cells lining the arterioles and AVS and by sensory and sympathetic fibers innervating these vascular elements. (Rice, Albrecht et al. 2015). Recent studies discovered that the NaV1.7 sodium channel plays a key role in spinal cord nociceptive and pruriceptive synaptic transmission. These studies reveal that NaV1.7 is a target for itch management and an anti- NaV1.7 antibody has therapeutic potential for suppressing pain and itch. (Lee, Park et al. 2014).

There is a need in the field for an effective method and medicament for treating pruritus and/or itch. Surprisingly, the present inventors have found that certain pyrone-indole compounds have inhibitory properties at Nav1.7 and TRPV1 channels and may be useful for the treatment of pruritus and itch. Such compounds are described in U.S. Patent Nos. 7,635,710, 8,242,163 and 8,569,355 and International Patent Specification Nos. WO2007/093880A2 and WO2007/093880A3 but not for use in treating itch or pruritus. The entire contents of the above-mentioned patents and literature references are incorporated herein by reference.
US2013/324583A1 discloses indolopyrones for use in treating or preventing diabetes, sleep disorders and/or neurodegenerative diseases but not for use in treating itch or pruritus.
EP0834317A1 and WO2014/144545A2 disclose indole derivatives for medical use, in particular in the treatment of pruritus.

### SUMMARY OF THE INVENTION

The present disclosure provides in one aspect, use of at least one pyrone-indole compound selected from compounds described in U.S. Patent Nos. 7,635,710, 8,242,163 and 8,569,355 and International Patent Specification Nos. WO2007/093880A2 and WO2007/093880A3, the entire contents of these references are incorporated herein by reference, in an effective amount, in the manufacture of a medicament, for treating itch and /or pruritus, in a patient suffering from dermatological and non-dermatological conditions leading to such symptoms, wherein the medicament additionally comprises at least one pharmaceutically acceptable diluent, preservative, antioxidant, solubilizer, emulsifier, adjuvant or carrier.

In another aspect, the present disclosure provides a pharmaceutical composition for use in treating a subject suffering from itch and/or pruritus said composition comprising an effective amount of at least one compound of this disclosure to the subject.

In still another aspect, the present disclosure provides a medicament for use in a patient suffering from dermatological and non-dermatological conditions leading to itch and/or pruritus. The medicament composition contains an effective amount of at least one compound of this disclosure and at least one additional therapeutic agent selected from UV protectants, hypnotics, sedatives, analgesics, minor tranquilizers, and anti-inflammatory drugs, in addition to at least one pharmaceutically acceptable diluent, preservative, antioxidant, solubilizer, emulsifiers adjuvant or carrier.

### BRIEF DESCRIPTION OF THE DRAWINGS

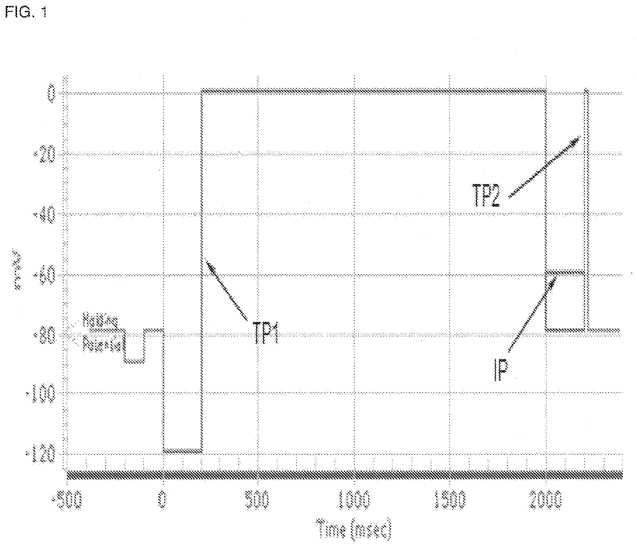

FIG. 1 shows a stimulus voltage pattern used for determining blocking effects on hNav1.x channels.

### DETAILED DESCRIPTION OF THE INVENTION

In one aspect, the present disclosure includes a pharmaceutical composition for use in treating a patient suffering from itch and/or pruritus said composition comprising an effective amount of at least one compound having the formula (I):

Ar-B-Ar' (I)

wherein:
-B- represents:

X-Y-Z-

wherein:
X represents -(CH₂)ₙ- (wherein n is 0-6), in which the alkyl moiety is linear or branched,
Y represents oxygen, sulphur, >NH or is absent;
Z represents >C = 0, or >0, or >COO or is absent;
wherein at least one of X, Y and Z must be present;
ring system Ar represents an indole nucleus: 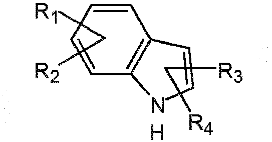
ring system Ar' represents an alpha-, beta- or gamma-pyrone nucleus: 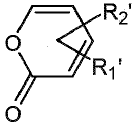 or 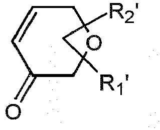 or 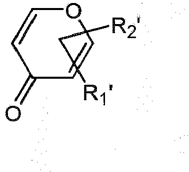 wherein each of the R₁₋₄ substitutes the ring systems Ar at any available position (including the N-position) and each of the R_{1'-2'} substitutes the ring system Ar' at any available position and wherein each of R₁₋₄ and R_{1'-2'} independently represents hydrogen, oxygen, halo, halo-C₁₋₅ alkyl, aryl, acyl, a C₅₋₇ heterocyclic group containing 1-3 hetero atoms independently selected from nitrogen, oxygen and sulphur; a C₆₋₈ heteroaryl group containing 1-3 hetero atoms independently selected from nitrogen, oxygen and sulphur; C₁₋₅ alkyl, C₂₋₅ alkenyl, C₂₋₅ alkynyl, aryl-C₁₋₅ alkyl, aryl-C₂₋₅ alkenyl, aryl-C₂₋₅ alkynyl, hydroxy-C₁₋₅ alkyl, nitro, amino, cyano, cyanamido, guanidino, amidino, acylamido, C₁₋₅ alkylamine, C₁₋₅ alkylamido, hydroxy, thiol, acyloxy, azido, C₁₋₅ alkoxy, carboxy, carbonylamido or styryl; wherein said arylalkyl, arylalkenyl, arylalkynyl, or styryl group optionally can be ring-substituted by one to four substituents independently selected from the group consisting of hydrogen, halo, halo-C₁₋₅ alkyl, aryl, a C₅₋₇ heterocyclic group containing from 1-3 heteroatoms independently selected from nitrogen, oxygen and sulphur; a heteroaryl group containing from 1-3 hetero atoms independently selected from nitrogen, oxygen and sulphur; C ₁₋₅ alkyl, C₂₋₅ alkenyl, C₂₋₅ alkynyl, aryl-C₂₋₅ alkenyl, aryl-C₂₋₅ alkynyl, hydroxy-C₁₋₅ alkyl, nitro, amino, cyano, cyanamido, guanidino, amidino, acylamido, hydroxy, thiol, acyloxy, azido, alkoxy, carboxy, carbonylamido, S-alkyl or alkylthiol; and either of R₃ or R₄ further can include or represent a bond to B;
wherein Ar is bonded to B at position 3 on the five-membered ring portion of the Ar ring, and Ar' can be bonded to B at any carbon on the Ar' ring not substituted by R_{1'} or R_{2'};
or a pharmaceutically acceptable salt, stereoisomer, or racemic mixture thereof, and at least one pharmaceutically acceptable diluent, preservative, antioxidant, solubilizer, emulsifier, gelling agent, adjuvant or carrier.

**As** used herein, "aryl" represents phenyl or naphthyl.

Also as used herein, reference to "a" compound, salt, stereoisomer, or racemic mixture of formula (I) is intended to encompass "one or more" such compounds, salts or stereoisomers. Furthermore, reference to a "compound" of formula (I), as in the discussion below of pharmaceutical formulations, is also intended to include a salt, stereoisomer, or racemic mixture of the compound.

In a preferred embodiment, X is -(CH₂)ₙ-, wherein n is any of 0-6 and preferably any of 1-6, Y is >NH or >O and Z is >CO.

Without prejudice to the generality of the compounds of the present invention, in a preferred embodiment of the compounds defined by formula (I), X is -(CH₂)₂-, Y is >NH or >O, Z is >C=O, Ar is an indole containing a bond, R₃ to X at position 3 of the indole ring, R₁ is methoxy on position 5 of the indole ring, each of R₂ and R₄ is hydrogen, Ar' is a gamma-pyrone bonded to Z at position 2 of the pyrone ring, R_{1'} is hydrogen or a hydroxy group at position 5 of the pyrone ring and R_{2'} is hydrogen or a carboxy group at position 6 of the gamma pyrone ring; or a pharmaceutically acceptable salt, stereoisomer, or racemic mixture thereof. In a second preferred embodiment, Ar is as defined above and Ar' is an alpha-pyrone ring bonded to Z at position 5 of the alpha-pyrone ring and R_{1'} and R_{2'} are hydrogens; or a pharmaceutically acceptable salt, stereoisomer, or racemic mixture thereof.

Suitable pharmaceutically acceptable salts of the compounds of formula (I) include salts which may, for example, be formed by mixing a solution of the compound with a solution of a pharmaceutically acceptable acid. Pharmaceutically acceptable acids include, but are not limited to hydrochloric acid, fumaric acid, maleic acid, succinic acid, acetic acid, citric acid, benzoic acid, tartaric acid, carbonic acid, phosphoric acid or sulfuric acid. Salts of amine groups may also comprise the quaternary ammonium salts in which the amino nitrogen atom carries an alkyl, alkenyl, alkynyl or aralkyl group. Where the compound carries an acidic group, for example a carboxylic acid group, the present invention also contemplates salts thereof, preferably non-toxic pharmaceutically acceptable salts thereof, such as the sodium, potassium and calcium salts thereof. Representative pharmaceutically acceptable salts include, yet are not limited to, acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

In some embodiments, the functional groups of the compounds of formula (I) useful in the invention can be modified to enhance the pharmacological utility of the compounds. Such modifications are well within the knowledge of a person of ordinary skill in the art and include, without limitation, esters, amides, ethers, N-oxides, and pro-drugs of the indicated compound of formula (I). Examples of modifications that can enhance the activity of the compounds of formula (I) include, for example, esterification such as the formation of C₁ to C₆ alkyl esters, preferably C₁ to C₄ alkyl esters, wherein the alkyl group is a straight or branched chain. Other acceptable esters include, for example, C₁ to C₇ cycloalkyl esters and arylalkyl esters such as benzyl esters. Such esters can be prepared from the compounds described herein using conventional methods well known in the art of organic chemistry.

It is understood that, in embodiments where the compounds of formula (I) useful in the invention have at least one chiral center, the compounds can exist as chemically distinct enantiomers. In addition, where a compound has two or more chiral centers, the compound can exist as diastereomers. All such isomers and mixtures thereof are encompassed within the scope of the indicated compounds of formula (I). Similarly, where the compounds possess a structural arrangement that permits the structure to exist as tautomers, such tautomers are encompassed within the scope of the indicated compound. Furthermore, in crystalline form, the compounds may exist as polymorphs; in the presence of a solvent, a compound may form a solvate, for example, with water or a common organic solvent. Such polymorphs, hydrates and other solvates also are encompassed within the scope of the invention as defined herein.

In one embodiment, the present disclosure includes a cream formulation for topical application comprising shea butter, coconut oil, and a therapeutically effective amount of a pyrone-indole derivative. The cream formulation may include N-[2-(1 H-indol-3-yl)-ethyl]-comanilamide. The concentration of the N-[2-(1 H-indol-3-yl)-ethyl]-comanilamide may be, e.g.,1-10%, 2-8%, 3-5%, or 3%.

A pharmaceutical composition useful in the methods of the invention can be administered to a subject by a variety of means depending, for example, on the type of pain to be treated, the compound to be included in the composition, and the history, risk factors and symptoms of the subject. The compounds of the invention may be administered by oral, parenteral (e.g., intramuscular, intraperitoneal, intravenous or subcutaneous injection, or implant), intrapulmonary (e.g., by inhalation), nasal, rectal, transbuccal, transdermal, or topical routes of administration. The compounds may also be administered by electrophoresis; topically in any acceptable form such as in drops, creams, gels or ointments; and by minipump or other implanted prolonged release device or formulation. "Prolonged release" refers to release of an active agent from a dosage form at a rate effective to achieve a therapeutic amount of the agent, or active metabolite thereof, locally or in the systemic blood circulation over a prolonged period of time. Release of the agent occurs over an extended period of hours, for example, over a period of at least 6 hours, over a period of at least 8 hours, over a period of at least 12 hours, or over a period of at least 24 hours. It is understood that different means of drug delivery can be combined in a method of the invention. As an example, intravenous administration on a first day can be combined with oral or topical dosing on a second and/or third day.

The term "subject" or "patient" as used herein, refers to a vertebrate, including but not limited to fish (such as commercially farmed fish, pet fish, etc.), amphibians (such as frogs, toads, pet amphibians, etc.), reptiles (such as snakes, lizards, turtles, pet reptiles, etc.), birds (such as chickens, turkeys, pet birds, etc.) and mammals (such as mice, rats, hamsters, rabbits, pigs, dogs, cats, horses, cows, sheep, goats, non-human primates, non-human mammals, pet non-human mammals, humans, etc.). In certain embodiments, the subject or patient is a mammal. In certain embodiments, the subject or patient is a mouse, a rat, a hamster, a rabbit, a pig, a dog, a cat, a horse, a cow, a sheep, a goat, a non-human primate, or a human (which may be included in embodiments of the invention individually or in any combination). In certain embodiments, the subject or patient is a human. In certain embodiments, the subject or patient is a non-human mammal.

Solid dosage forms for oral administration include capsules, tablets, pills, powders and granules. In such solid dosage forms, the active compound is admixed with at least one inert pharmaceutically acceptable carrier such as sucrose, lactose, or starch. Such dosage forms can also comprise, as is normal practice, additional substances other than inert diluents, e.g., lubricating agents such as magnesium stearate. Illustrative of the adjuvants which may be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. In the case of capsules, tablets and pills, the dosage forms may also comprise buffering agents. When the dosage unit form is a capsule, it may contain, in addition to materials of the above type, a liquid carrier such as a fatty oil. Various other materials can be present as coatings or to otherwise modify the physical form of the dosage unit. Tablets and pills can additionally be prepared with enteric coatings and tablets may be coated with shellac, sugar or both.

Liquid dosage forms for oral administration include pharmaceutically acceptable emulsions, solutions, suspensions, syrups and elixirs containing inert diluents commonly used in the art, such as water. Besides such inert diluents, compositions can also include adjuvants, such as wetting agents, emulsifying and suspending agents, and sweetening, flavoring, and perfuming agents. A syrup or elixir may contain the active compound, sucrose as a sweetening agent, methyl and propyl parabens as preservatives, a dye and a flavoring such as cherry or orange flavor.

Preparations according to this invention for parenteral administration include sterile aqueous or non-aqueous solutions, suspensions, or emulsions. Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc., or a synthetic fatty vehicle like ethyl oleate or the like. Buffers, preservatives, antioxidants and the like may be incorporated as required. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in the form of sterile solid compositions that can be dissolved in sterile water, or some other sterile injectable medium immediately before use.

The dosage of active agent in compositions of this disclosure can vary, provided that a therapeutic amount is administered. Such therapeutic amount generally is the minimum dose necessary to achieve the desired therapeutic effect, which can be, for example, that amount roughly necessary to reduce the itching to tolerable levels. Desirably the active agent is administered to a patient (human or animal) in need of such treatment in dosages that will provide optimal pharmaceutical efficacy. The selected dosage depends upon the nature and severity of the disease or disorder to be treated, desired therapeutic effect, the route of administration, and the duration of treatment. The dose will vary from patient to patient depending on the nature and severity of the disease, the patient's weight, special diets then being followed by the patient, concurrent medication, the bioavailability of the compound upon administration and other factors which those skilled in the art will recognize. Therapeutic doses are generally in the range of 0.1-1000 mg/day and can be, for example, in the range of 0.1-500 mg/day, 0.5-500 mg/day, 0.5-100 mg/day, 0.5-50 mg/day, 0.5-20 mg/day, 0.5-10 mg/day or 0.5-5 mg/day, with the actual amount to be administered determined by a physician taking into account the relevant circumstances including the severity of the itching, the age and weight of the patient, the patient's general physical condition, the cause of itching and the route of administration. In some embodiments, the therapeutically effective amount comprises a dosage of 0.10 mg, 0.15 mg, 0.20 mg, 0.25 mg, 0.5 mg, 0.75 mg, 1 mg, 2 mg, 2.5 mg, 3 mg, 4 mg, 5 mg, 6 mg, 7 mg, 8 mg, 9 mg, 10 mg, 15 mg, 20 mg, 25 mg, or 30 mg one or more times a day. As a non-limiting example, the compounds of the present invention may be administered by repeated dosing or continuous dosing over a period of at least three days, or for example, over three days, four days, five days, six days, seven days, eight days, nine days or ten days. As a further example, the compounds can be administered multiple times a day, such as twice per day, three times per day, four times per day or more.

**In** some embodiments, the dose of the compound is sufficient to reduce itch and/or pruritus by at least 30%. In other embodiments, itch and/or pruritus are reduced by at least 40%, 50%, 60%, 70%, 80%, 90%, or 100%.

**A** pharmaceutical composition useful in the invention includes the active compound (i.e. a compound of formula (I)) and further can include, if desired, an excipient such as a pharmaceutically acceptable carrier or a diluent, which is any carrier or diluent that has substantially no long term or permanent detrimental effect when administered to a subject. Such an excipient generally is mixed with active compound, or permitted to dilute or enclose the active compound. A carrier can be a solid, semi-solid, or liquid agent that acts as an excipient or vehicle for the active compound. Examples of pharmaceutically acceptable carriers and diluents include, without limitation, water, such as distilled or deionized water; saline; and other aqueous media. It is understood that the active ingredients can be soluble or can be delivered as a suspension in the desired carrier or diluent.

In certain embodiments, a pharmaceutical formulation for use according to the present disclosure is characterized by at least one of the following features:
i. it is adapted to be administered by oral, parenteral (e.g., intramuscular, intraperitoneal, intravenous or subcutaneous injection, or implant), intrapulmonary (e.g., by inhalation), nasal, rectal, transbuccal, transdermal, or topical routes of administration and can be formulated in dosage forms appropriate for each route of administration;
ii. it is in unit dosage form, each unit dosage in an effective amount;
iii. it is a prolonged release formulation.
iv. it is in a depot form which will release the compound slowly in the body, over a preselected time period;
v. it is an ointment, cream, foam or spray intended for topical use;
vi. it optionally includes at least one additional therapeutic agent selected from UV protectants, analgesics, minor tranquilizers, and anti- inflammatory drugs.

The present disclosure includes a composition containing at least one compound in an amount effective in treating itch and/or pruritus in a patient suffering from dermatological and non-dermatological conditions leading to such symptoms and at least one pharmaceutically acceptable diluent, preservative, antioxidant, solubilizer, emulsifiers adjuvant or carrier. More preferably, the composition is further characterized by at least one of the features (i), (ii), (iii), (iv) (v) and (vi), set forth above.

In the composition with which the present invention is concerned, the pharmaceutically acceptable diluents, preservatives, solubilizers, emulsifiers, adjuvants and carriers are those acceptable for pharmaceutical formulations. The pharmaceutical composition can include, if desired, one or more agents such as emulsifying agents, wetting agents, sweetening or flavoring agents, tonicity adjusters, preservatives, buffers or anti-oxidants. Tonicity adjustors useful in a pharmaceutical composition include salts such as sodium chloride, potassium chloride, mannitol or glycerin and other pharmaceutically acceptable tonicity adjustors. Preservatives useful in the pharmaceutical compositions of the invention include, without limitation, benzalkonium chloride, chlorobutanol, thimerosal, phenylmercuric acetate, and phenylmercuric nitrate. Various buffers and means for adjusting pH can be used to prepare a pharmaceutical composition, including, but not limited to, acetate buffers, citrate buffers, phosphate buffers and borate buffers. Similarly, anti-oxidants useful in the pharmaceutical compositions of the invention are well known in the art and include, for example, sodium metabisulfite, sodium thiosulfate, acetylcysteine, butylated hydroxyanisole and butylated hydroxytoluene. It is understood that these and other substances known in the art of pharmacology can be included in a pharmaceutical composition useful in the invention.

For oral administration, the medicament may be utilized as e.g. tablets, capsules, emulsions, solutions, syrups or suspensions. For parenteral administration, the medicament may be utilized in the form of ampoules, or otherwise as suspensions, solutions or emulsions in aqueous or oily vehicles or patch. For ectopic use (topical administration) the medicament may be utilized in the form of ointment, cream, gel, foam, solution, aerosol or spray. The medicament may be water-based (aqueous) or organic-based. The need for suspending, stabilizing and/or dispersing agents will of course take account of the fact of the solubility or otherwise of the active compounds, in the vehicles which are used in particular embodiments. The medicament may additionally contain e.g. physiologically compatible preservatives and antioxidants. The medicament may also be utilized as suppositories with conventional suppository bases such as cocoa butter or other glycerides.

As described above, the present disclosure includes co-administering the compounds described above at least one compound selected from such compounds described in US Patent Nos. US 7635710, US 8242163 and US 8569355 and International Patent Specification No. WO2007093880A2 and WO2007093880A3, may be administered in conjunction with (i.e. simultaneously, separately or sequentially) other compounds which are known in the art to be useful for protecting the skin and for alleviating itch by reducing anxiety, improving sleep and reducing pain, including e.g., at least one additional therapeutic agent selected from hypnotics (benzodiazepines as well as non- benzodiazepines and melatonin agonists), sedatives, analgesics, minor tranquilizers, and anti-inflammatory drugs. Examples of such additional therapeutic agents are brotizolam, buspirone, diazepam, diphenhydramine, doxepin, fluvoxamine, clonidine, zolpidem, zopiclone, Melatonin, UV protectants (p-aminobenzoic acid, menthyl anthranilate, octyl methoxycinnamate, titanium dioxide, etc.) and pharmaceutically acceptable salts and combinations thereof.

The additional therapeutic agent may be e.g., an anti-inflammatory corticosteroid, such as dexamethasone or melatoninergic agents, at least one compound selected from melatonin, other melatonergic agents, melatonin agonists and/or antagonists, may be administered in conjunction with the use of physical methods such as with light therapy or electrical stimulation, e.g. scheduling bright light administration, ordinary-intensity light exposure, or exposure to dim-light or darkness.

It is understood that the instant disclosure encompasses compounds which inhibit, inter alia, channels involved in itch response. It is further understood that the instant disclosure encompasses compounds which are either selective or non-selective for such channels. In some embodiments, the compounds of the invention are highly selective for a channel involved in itch response, and are characterized as having a potency of less than 900nM, 800nM, 700 nM, 600 nM, 500 nM, 400 nM, 300 nM, 200 nM, 100 nM, 50 nM, 10 nM or 1 nM.

The present invention also provides a method of screening for effective agents that reduce itching, comprising identifying a compound believed to have having anti-itch activity, contacting the compound with a receptor involved in the itch response, and determining whether the compound has anti-itch activity.

Many modifications and variations of this invention can be made without departing from its spirit and scope, as will be apparent to those skilled in the art. The specific embodiments described herein are offered by way of example only, and the invention is to be limited only by the terms of the appended claims, along with the full scope of equivalents to which such claims are entitled. All patents and publications cited herein are incorporated by reference.

The invention will be illustrated by the following Examples.

### EXAMPLES

### Example 1

### Synthesis of N-[2-(1 H-indol-3-yl)-ethyl]-comanilamide

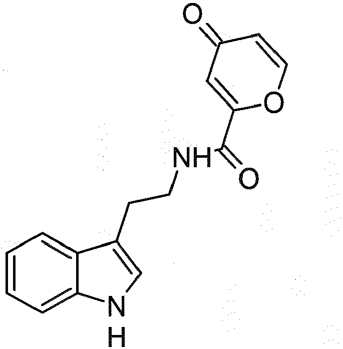

### Reaction Scheme for the Synthesis of N-[2-(1 H-indol-3-yl)-ethyl]-comanilamide

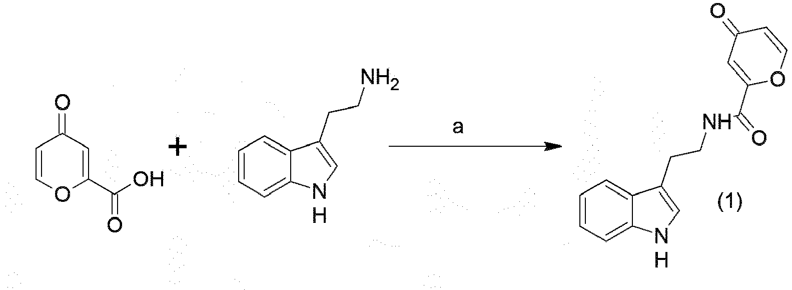

a: DME, Py, TEA, DCC, NHS

### General Procedure for the Synthesis of N-[2-(1H-indol-3-yl)-ethyl]-comanilamide

In a four necked round bottom flask (100 mL), tryptamine (0.85 g, 5.3 mmol)) was dissolved in DME (40 mL) and Py (0.9 mL) was added. The mixture was stirred 30 minutes at room temperature then comanic acid (0.68 g, 4.8 mmol) was added, followed by NHS (0.61 g, 5.3 mmol), DCC (1.1 g, 5.3 mmol), TEA (0.7 mL, 56.1 mmol); the mixture was stirred overnight, then checked by LC-MS. The brown suspension was filtered on a gooch, washing the solid with water (100 mL). The aqueous phase was extracted with CH₂Cl₂ (3X100 mL), washed with brine (2x10 mL), dried over MgSO₄, filtered and evaporated under reduced pressure. The obtained sticky solid was suspended and stirred in MTBE (20 mL) for 30 minutes. The solid was filtered and washed with CH₂Cl₂ to give 0.48 g (4.8 mmol, yield 36%) of product (1) as a light brown solid (LC-MS assay >95%).

Experimental data for N-[2-(1H-indol-3-yl)-ethyl]-comanilamide
MS: m/z = 283 [M+H]+
TLC: (Cy/EtOAc 1:9) Rf = 0.3
FTIR (cm-1): 3330, 2925, 2851, 2353, 2116, 1240, 937, 741.
1H NMR (DMSO): 2.91 (m, 2H); 3.55 (m, 3H); 6.42 (m, 1H); 6.79 (m, 1H); 6.99 (m, 1H); 7.07 (m, 1H); 7.19 (m, 1H); 7.34 (m, 1H); 7.58 (m, 1H); 8.21 (m, 1H); 9.05 (m, 1H); 10.83 (bs, 1H).

### Example 2

### Synthesis of N-[2-(5-methoxy-indol-3-yl)-ethyl]-comanilamide

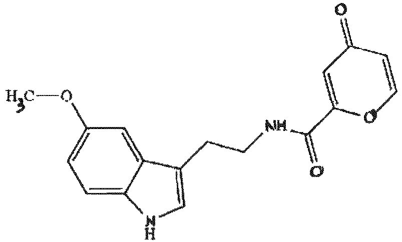

Reaction Scheme for the Synthesis of N-[2-(5-methoxy-indol-3-yl)-ethyl]-comanilamide 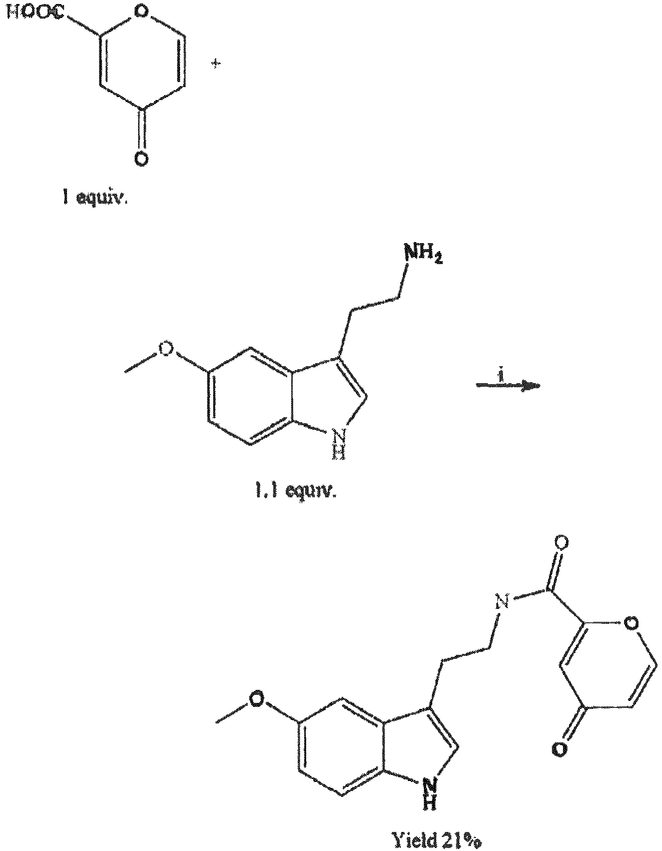 i. DMF, HOBt, 1.1 equiv., EDC 1.1 equiv., Net₃ 2.5 equiv., r.t., 6h.

### General Procedure for the Synthesis of N-[2-(5-methoxy-indol-3-yl)-ethyl]-comanilamide

Under an argon atmosphere, a 100 ml three-necked flask round-bottom flask was charged with comanic acid (500 mg, 1 equiv.) and 5-methoxytryptamine (760 mg, 1.1 equiv.), dissolved in DMF (25 ml), and brought to 0° C by means of an ice-bath. HOBt (1-hydroxybenxotriazole monohydrate, 530 mg, 1.1 equiv.), EDC (1-(3-dimethylaminopropyl)-3-ethylcarbodiimide hydrochloride, 750 mg, 1.1 equiv.) and triethylamine (1.25 ml, 2.5 equiv.) were then added under magnetic stirring. The mixture was stirred for an additional 15 minutes at 0° C and subsequently allowed to react for 6 h at room temperature. The reaction course was followed by HPLC-MS. Water (50 ml) was then added and the mixture was extracted with dichloromethane (3×50 ml). The combined organic phases were dried over Na₂SO₄ and the solvent was removed by rotary evaporation. The crude was then chromatographed over a silica gel column by eluting with dichloromethane/methanol 95/5. The product was recovered as a bright yellow solid (235 mg, yield 21%).

Experimental data for N-[2-(5-methoxy-indol-3-yl)-ethyl]-comanilamide MS (ESI POS): 313 (M+H), 330 (M+H2O), 335 (M+Na), 376 (M+Na+CH3CN)
HPLC assay: 98%
1H NMR (DMSO-d6, 400 MHz) δ 2.88-2.92 (m, 2H, CH2CH2NH), 3.48-3.53 (m, 2H, CH2CH2NH), 3.75 (s, 3H, OCH3), 6.42 (dd, J1=2.3 Hz, J2=5.9 Hz, 1H, CH=CH), 6.71 (dd, J1=2.1 Hz, J2=8.8 Hz, 1H, aromatic H), 6.78 (d, J=2.3 Hz, 1H, aromatic H), 7.04 (d, J=2.3 Hz, 1H, CH), 7.13 (d, J=2.1 Hz, 1H, aromatic H), 7.22 (d, J=8.8 Hz, 1H, aromatic H), 8.21 (d, J=5.9 Hz, 1H, CH=CH—CO), 9.04 (br t, J=5.8 Hz, 1H, CH2CH2NH), 10.65 (br s, 1H, NH).

### Example 3

### Synthesis of 2-methyl-4-oxo-4H-pyran-3-yl [2-(5-methoxy-1H-indol-3-yl)ethyl]carbamate:

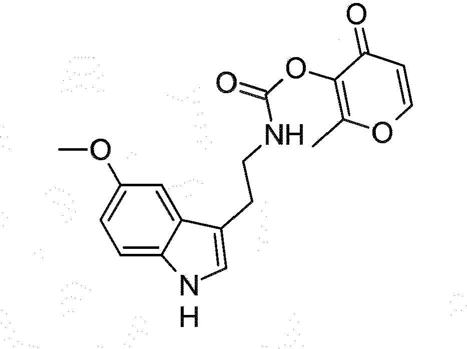

### Reaction Scheme for the Synthesis of 2-methyl-4-oxo-4H-pyran-3-yl [2-(5-methoxy-1H-indol-3-yl)ethyl]carbamate

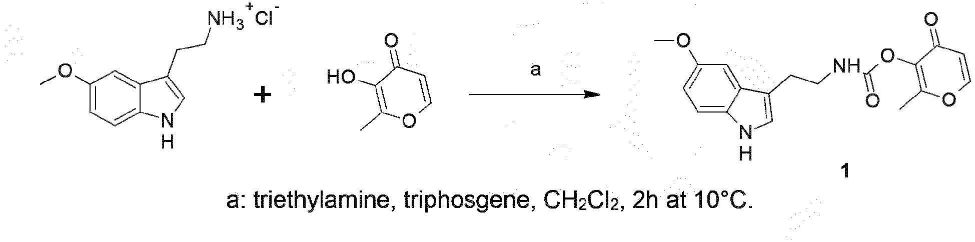

1: 2-methyl-4-oxo-4H-pyran-3-yl [2-(5-methoxy-1H-indol-3-yl)ethyl]carbamate

To a suspension of 5-methoxytryptamine hydrochloride (1g, 0.0044mol) in CH₂Cl₂ (7ml) triethylamine (1.8ml, 0.0132mol) was added and the mixture stirred for 30' at room temperature. After the addition of maltol (0.5g, 0.0044mol) the reaction mixture was cooled in an ice-water bath then triphosgene (0.4g, 0.0015mol) in CH₂Cl₂ (1ml) was added, keeping the temperature below 10°C, and stirred for 2 h at the same temperature. The mixture was evaporated under reduced pressure to give an oily residue that was purified by flash chromatography (EtOAc-Cy, 80:20 to ETOAc 100) giving 500mg of pale-yellow solid. This solid was purified by a second flash chromatography (VersaPak C18, Spherical, 23x53mm, H₂O-MeOH, 6:4) yielding 220mg of white foam that was washed with MTBE to give 150mg of 1 as a white solid. Rf: 0,11 (EtOAc-Cy, 8:2); 0,47 (reversed phase; MeOH-H₂O, 6:4)
HPLC: tr = 5.5 Assay > 95%
MS (ESI+) m/z=343 [M+H]+
FTIR (cm-1): 3855; 3751; 3328; 2938; 2270; 1735; 1656; 1578; 1526; 1485; 1438; 1364; 1236; 1183; 1072; 1034; 923; 834; 798; 637; 566; 432.

1H-NMR(CDCl3): 2.25 (s, 3H), 3.01 (t, 2H), 3.57 (q, 2H), 3.86 (s, 3H), 5.29 (t, 1H), 6.39 (d, 1H), 6.85-6.88 (m, 1H), 7.05 (d, 1H), 7.13 (d, 1H), 7.26 (d, 1H), 7.65 (d, 1H), 8.05 (bs, 1H).

### Example 4

Formalin injected in the nape of the neck was previously reported to elicit significant hindlimb scratching in rats and mice, and formalin as well as pruritogens elicited enhanced scratching in mice lacking a population of inhibitory spinal interneurons, suggesting that formalin-evoked nocifensive behavior may reflect itch rather than, or in addition to, pain (Akiyama, Carstens et al. 2010). The anti-pruritus activity of pyrone-indole compounds were assessed using this model.

Method. The noxious stimulus is an injection of dilute formalin under the skin of the dorsal surface of the right hindpaw. The response is the amount of time the animals spend licking the injected paw 5-10 minutes (early phase), or 20-30 minutes (late phase) after the injection of formalin. Mice (N=8 per group) were injected intraperitoneally with test compound (100 mg/kg), or morphine (4 mg/kg), or vehicle. Thirty minutes later, formalin (20 µl 2.5 % in water) was injected by the subplantar route into the right hindpaw. Hindpaw licking time was recorded in consecutive 5 minute-intervals during the first 10 minutes (early phase), and from 15 to 35 minutes (late phase) after formalin injection.

Results. The compound N-[2-(1H-indol-3-yl)-ethyl]-comanilamide resulted in inhibition of the licking response by 57% in the early and 90% in the late phase, both statistically different from vehicle response. The compound N-[2-(5-methoxy-indol-3-yl)-ethyl]-comanilamide resulted in inhibition of the licking response by 38% in the early and 62% in the late phase, both statistically different from vehicle response. The compound 2-methyl-4-oxo-4H-pyran-3-yl [2-(5-methoxy-1H-indol-3-yl)ethyl]carbamate resulted in inhibition of the licking response by 54% in the early and 86% in the late phase, both statistically different from vehicle response.

Conclusions. These results show beneficial effects of N-[2-(1H-indol-3-yl)-ethyl]-comanilamide, N-[2-(5-methoxy-indol-3-yl)-ethyl]-comanilamide and 2-methyl-4-oxo-4H-pyran-3-yl [2-(5-methoxy-1H-indol-3-yl)ethyl]carbamate on itch induced by formalin injection in the mouse. N-[2-(1H-indol-3-yl)-ethyl]-comanilamide has potential anti-itching effects in pruritus.

### Example 5

### Effects of N-[2-(1H-indol-3-yl)-ethyl]-comanilamide, N-[2-(5-methoxy-indol-3-yl)-ethyl]-comanilamide and 2-methyl-4-oxo-4H-pyran-3-yl [2-(5-methoxy-1H-indol-3-yl)ethyl]carbamate on Nav1.x Channels Expressed in Mammalian Cells

Method. A block of hNav1.x channels was measured using the stimulus voltage pattern shown in FIG. 1; voltage potentials are indicated in Table 2, below. The pulse pattern repeated twice: before and 5 minutes after N-[2-(1H-indol-3-yl)-ethyl]-comanilamide, N-[2-(5-methoxy-indol-3-yl)-ethyl]-comanilamide and 2-methyl-4-oxo-4H-pyran-3-yl [2-(5-methoxy-1H-indol-3-yl)ethyl]carbamate addition and peak current amplitudes at 3 test pulses was measured (ITP1 and ITP2).

Results. N-[2-(1H-indol-3-yl)-ethyl]-comanilamide, N-[2-(5-methoxy-indol-3-yl)-ethyl]-comanilamide and 2-methyl-4-oxo-4H-pyran-3-yl [2-(5-methoxy-1H-indol-3-yl)ethyl]carbamate blocking effects on hNav1.x channels (Table 3) showing preferential inhibition of Nav1.7 channels by N-[2-(1H-indol-3-yl)-ethyl]-comanilamide.

Conclusions. These results show inhibition of Nav1.7 channels that mediate itch response using the compounds of this disclosure.

**Table 2**

| **Vo ltage-protocol parameters for hNav1.x channels** | | | | | | |
|---|---|---|---|---|---|---|
| **Channel** | | **Holding Potential (mV)** | **200-ms Pre-Pulse Potential (mV)** | **1800-ms Test Pulse 1 (mV)** | **200-ms Inter Pulse (mV)** | **50-ms Test Pulse 2 Potential (mV)** |
| Nav1.1-1.7 | | -80 | -120 | 0 | -60 | 0 |
| Nav1.8/beta3 | | -80 | -120 | 20 | -60 | 20 |

**Table 3**

| Test Article ID | Type of the block | IC50, mM | | | | | | | |
|---|---|---|---|---|---|---|---|---|---|
| | | Nav1 .1 | Nav1 .2 | Nav1 .3 | Nav1 .4 | Nav1 .5 | Nav1 .6 | Nav1 .7 | Nav1.8 /b3 |
| | Tonic | >100 | >100 | >100 | >100 | >100 | >100 | >100 | >100 |
| N-[2-(1H-indol-3-yl)-ethyl]-comanila mide | | | | | | | | | |
| | Inactiva ted State | 76.0 | 47.6 | 58.7 | >100 | 78.6 | 94.1 | 18.3 | 94.1 |
| | | | | | | | | | |
| N-[2-(5-methoxy-indol-3-yl)-ethyl]-comanila mide ted | Inactiva State | | | 71.6 | | | | 87.0 | >100 |
| | | | | | | | | | |

| Test Article ID | Type the block | of IC50, mM | | | | | | | |
|---|---|---|---|---|---|---|---|---|---|
| | | Nav 1.1 | Nav 1.2 | Nav 1.3 | Nav 1.4 | Nav 1.5 | Nav 1.6 | Nav 1.7 | Nav1.8 /b3 |
| | Tonic | >100 | >100 | >100 | >100 | >100 | >100 | >100 | >100 |
| 2-methyl-4-oxo-4 H-pyran-3-yl [2-(5-methoxy-1 H-indol-3-yl)ethyl]carb amate | Inactiva ted State | | | >100 | | | | 133. 2 | >100 |

### Example 6

### Effect of N-[2-(1 H-indol-3-yl)-ethyl]-comanilamide, N-[2-(5-methoxy-indol-3-yl)-ethyl]-comanilamide and 2-methyl-4-oxo-4H-pyran-3-yl [2-(5-methoxy-1H-indol-3-yl)ethyl]carbamate on TRPV1 channels.

Method. The ability of N-[2-(1H-indol-3-yl)-ethyl]-comanilamide, N-[2-(5-methoxy-indol-3-yl)-ethyl]-comanilamide and 2-methyl-4-oxo-4H-pyran-3-yl [2-(5-methoxy-1 H-indol-3-yl)ethyl]carbamate to act as antagonists of TRPV1 was evaluated with a calcium influx assay. The signal elicited in the presence of the positive control agonist (0.1 µM capsaicin) was set to 100% and the signal in the presence of the antagonist (0.1 µM capsaicin + 3µM ruthenium red) was set to 0. Values were considered significant if the test article mean was three or more standard deviations away from the positive control agonist mean (i.e., greater than 37.74% inhibition).

Results. N-[2-(1 H-indol-3-yl)-ethyl]-comanilamide, N-[2-(5-methoxy-indol-3-yl)-ethyl]-comanilamide and 2-methyl-4-oxo-4H-pyran-3-yl [2-(5-methoxy-1 H-indol-3-yl)ethyl]carbamate (10 µM) had a significant inhibitory effect (i.e. ≥37.74% inhibition) on TRPV1 mediated calcium influx (Table 2).

| Test Article | Test conc (µM) | Normalized % Activation | SD | Normalized % inhibition | SD |
|---|---|---|---|---|---|
| N-[2-(1 H-indol-3-yl)-ethyl]-comanilamide | 0.1 | 23.07 | 4.66 | 9.88 | 25.02 |
| | 0.5 | 11.93 | 1.79 | 2.44 | 7.90 |
| | 1 | 10.51 | 6.61 | 31.12 | 1.89 |
| | 5 | 8.71 | 6.00 | 26.08 | 10.09 |
| | 10 | 10.13 | 1.05 | 37.74 | 5.57 |

| Test Article | Test conc (µM) | Normalized % Activation | SD | Normalized % inhibition | SD |
|---|---|---|---|---|---|
| N-[2-(5-methoxy-indol-3-yl)-ethyl]-comanilamide | 0.1 | 18.12 | 4.10 | 15.21 | 17.68 |
| | 0.5 | 11.95 | 1.38 | 1.19 | 3.64 |
| | 1 | 14.04 | 4.20 | 36.36 | 4.62 |
| | 5 | 12.92 | 5.98 | 35.39 | 7.47 |
| | 10 | 13.67 | 1.52 | 42.59 | 5.39 |

| Test Article | Test conc (µM) | Normalized % Activation | SD | Normalized % inhibition | SD |
|---|---|---|---|---|---|
| 2-methyl-4-oxo-4H-pyran-3-yl [2-(5-methoxy-1 H-indol-3-yl)ethyl]carbamate | 0.1 | 12.65 | 5.35 | -1.78 | 10.57 |
| | 0.5 | 9.58 | 3.56 | 23.56 | 5.32 |
| | 1 | 12.16 | 6.56 | 45.10 | 9.63 |
| | 5 | 5.75 | 2.03 | 44.60 | 5.13 |
| | 10 | 12.26 | 0.81 | 41.46 | 6.61 |

Conclusions. The compounds of the invention are able to inhibit TRPV1 channels that are involved in itch response.

### Example 7

### Preparation of a topical N-[2-(1 H-indol-3-yl)-ethyl]-comanilamide cream.

A cream formulation for topical application of N-[2-(1H-indol-3-yl)-ethyl]-comanilamide was prepared by heating 50g shea butter, 50g coconut oil in a mason jar until the oils melt and added 3g (3%) N-[2-(1 H-indol-3-yl)-ethyl]-comanilamide until it is dissolved in the oils. The cream was cooled and subsequently whipped on high speed mixer for several minutes for softer texture.

### Example 8

### Analgesic/Anti-pruritus Effects in Humans

The anti-pruritus effect of N-[2-(1 H-indol-3-yl)-ethyl]-comanilamide was tested using the UVB sunburn model (R. Rolke et al. (2006), Quantitative sensory testing in the German Research Network on neuropathic pain (DFNS): standardized protocol and reference values, Pain, 123, 231-243) used as an art-accepted surrogate model of itch (Andersen et al., Acta Derm Venereol. 2015; 95(7):771-7. Human surrogate models of histaminergic and non-histaminergic itch.).

Twenty four young male subjects (18 - N-[2-(1 H-indol-3-yl)-ethyl]-comanilamide in escalated doses (300mg-900mg) and 6 in the placebo group), were exposed to three times the minimal erythema dose (MED) of UVB light on an approximately 4 x 4 cm area on the upper part of the leg in the morning of Day (-1) pre-dose on Day 1 and 2.5 hours post - dose on the UVB-irradiated area.

UVB irradiation causes skin inflammation which is noxious and elicits pronounced inflammation related temperature and mechanical hyperalgesia at the UVB-irradiated site. This hyperalgesia peaks at about 20-24 hours post irradiation after which it slowly subsides. Quantitative sensory testing (QST) assesses characteristic sensory patterns in pain and pruritus models.

The UVB irradiated skin was obtained by irradiating the marked area (approximately 4 x 4 cm) of the skin of the upper part of the right leg by three times the MED as determined during the screening. This was done approximately 20 hours prior to dosing.

Cold pain thresholds (CPT) were determined. The mean threshold temperature of five consecutive measurements was calculated. All thresholds were obtained with ramped stimuli (1 C/s) that were terminated when the subject pressed a button.

Mechanical detection threshold (MDT) to von Frey hairs was assessed by using a standardized set of modified von Frey hairs (Optihair; Marstock Nervtest, Marburg, Germany) exerting forces between 0.25 and 512 mN (rounded tip, 0.5 mm in diameter). Thresholds were determined by the method of limits with the geometric mean of a series of 5 ascending and descending stimulus intensities.

The results were as follows:
**Cold pain thresholds levels, pre and post sunburn and 2.5h post N-[2-(1 H-indol-3-yl)-ethyl]-comanilamide administration.**

| CPT (°C) | Pre sunburn | Post sunburn | 2.5h Post Drug Administration |
|---|---|---|---|
| 600mg | 5.55 | 16.03 | 12.75 |
| Placebo | 13.54 | 16.85 | 15.28 |

**Change from baseline of MDT (feel sensation) (mN) post sunburn and 2.5h post N-[2-(1H-indol-3-yl)-ethyl]-comanilamide administration.**

| ***MDT feel sensation (mN)*** | 300mg | 600mg | 900mg | Placebo |
|---|---|---|---|---|
| Post sunburn - **Change from Baseline*** | -10.92 | -3.52 | -7.24 | -11.57 |
| 2.5h post Drug | -12.13 | -0.017 | 0.84 | -8.57 |
| administration - **Change from Baseline*** | | | | |

| | | | | |
|---|---|---|---|---|
| *Baseline - Day (-1) before the sunburn. | | | | |

Conclusions. These results clearly demonstrated the analgesic/anti-pruritus effects of N-[2-(1H-indol-3-yl)-ethyl]-comanilamide in human testing, with a peak response at 2.5h post dosing.

Various embodiments of the invention comprise:
1. A pharmaceutical composition for use in treating a subject suffering from itch or pruritus, said composition comprising an effective amount of at least a compound having the formula

   Ar-B-Ar' (I)

   wherein:
   -B- represents:

   -X-Y-Z-,

   wherein:
   X represents -(CH₂)ₙ (wherein n is 0-6);
   Y represents oxygen, sulphur, >NH or is absent;
   Z represents >C=O, >O or >COO or is absent; and wherein at least one of X, Y and
   Z must be present;
   Ar represents an indole nucleus ring system: 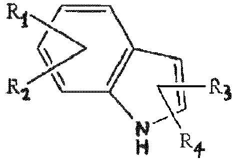
   Ar' represents an alpha-, beta- or gamma-pyrone nucleus ring system: 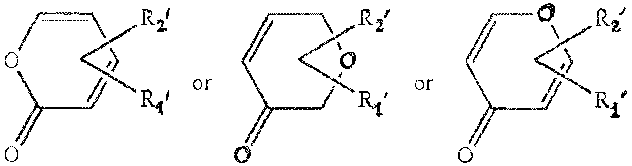 wherein each of R₁₋₄ substitutes the ring system Ar at any available position (including the N-position) and each of R_{1'}-R_{2'} substitutes the ring system Ar' at any available position and wherein each of R₁₋₄ and R_{1'-2'} independently represents hydrogen, oxygen, halo, halo-C₁₋₅ alkyl, aryl, acyl, a C₅₋₇ heterocyclic group containing 1-3 hetero atoms independently selected from nitrogen, oxygen or sulphur; a C₆₋₈ heteroaryl group containing 1-3 hetero atoms independently selected from nitrogen, oxygen or sulphur, C₁₋₅ alkyl, C₂₋₅ alkenyl, C₂₋₅ alkynyl, aryl- C₁₋₅ alkyl, aryl- C₂₋₅ alkenyl, aryl-C₂₋₅ alkynyl, hydroxy-C₁₋₅ alkyl, nitro, amino, cyano, cyanamide, guanidino, amidino, acylamido, C₁₋₅ alkylamine, C₁₋₅ alkylamido, hydroxy, thiol, acyloxy, azido, C₁₋₅ alkoxy, carboxy, carbonylamido or styryl; wherein said arylalkyl, arylalkenyl, aralalkynyl, or styryl group optionally can be ring-substituted by one to four substituents independently selected from the group consisting of hydrogen, halo, halo-C₁₋₅ alkyl, aryl, a C₅₋₇ heterocyclic group containing 1-3 hetero atoms independently selected from nitrogen, oxygen and sulphur; a heteroaryl group containing 1-3 hetero atoms independently selected from nitrogen, oxygen and sulphur; C₁₋₅ alkyl, C₂₋₅ alkenyl, C₂₋₅alkynyl, aryl-C₁₋₅ alkyl, aryl-C₂₋₅ alkenyl, aryl-C₂₋₅ alkynyl, hydroxy-Ci-s alkyl, nitro, amino, cyano, cyanamido, guanidino, amidino, acylamido, hydroxy, thiol, acyloxy, azido, aikoxy, carboxy, carbonylamido, S-alkyl or alkylthiol;
   and either of R₃ or R₄ further can include or represent a bond to B;
   wherein Ar is bonded to B at position 3 on the five-membered ring portion of the Ar ring and Ar' can be bonded to B at any carbon on the Ar' ring not substituted by R_{1'} and R_{2'}; or a salt, stereoisomer, or racemic mixture thereof, and at least one pharmaceutically acceptable diluent, preservative, antioxidant, solubilizer, emulsifier, gelling agent, adjuvant or carrier;
   wherein the pharmaceutical composition:
      (i) is adapted for oral, rectal, parenteral, transbuccal, intrapulmonary (e.g. by inhalation) transdermal or topical administration;
      (ii) is in unit dosage form, each unit dosage comprising an effective amount of said compound;
      (iii) is a prolonged release formulation;
      (iv) is in a depot form which will release the compound slowly in the body, over a preselected time period;
      (v) is an ointment, cream, gel, foam, emulsion, oil, solution, or spray suitable for topical use;
      (vi) further comprises at least one additional therapeutic agent selected from a UV protectant, an analgesic, a tranquilizer, a vasoconstrictor, a vasodilator, and an anti-inflammatory agent; and/or
      (vii) is a solid dosage form for oral administration.
2. The pharmaceutical composition of embodiment 1, wherein the composition is an oral dosage form selected from capsules, tablets, pills, powders or granules.
3. The pharmaceutical composition of embodiment 1 or 2, wherein the compound of formula I is admixed with at least one inert pharmaceutically acceptable carrier selected from sucrose, lactose, or starch.
4. The pharmaceutical composition of any of embodiments 1 to 3, wherein the composition further comprises one or more lubricating agents.
5. The pharmaceutical composition of any of embodiments 1 to 4, wherein the composition comprises magnesium stearate.
6. The pharmaceutical composition of any of embodiments 1 to 5, wherein the composition comprises one or more adjuvants.
7. The pharmaceutical composition of any of embodiments 1 to 6, wherein the composition comprises gum tragacanth, acacia, corn starch or gelatin.
8. The pharmaceutical composition of any of embodiments 1 to 7, wherein the composition comprises at least one of an excipient, a disintegrating agent, a lubricant, a sweetening agent, and a flavoring agent.
9. The pharmaceutical composition of embodiment 8, wherein the excipient is microcrystalline cellulose.
10. The pharmaceutical composition of embodiment 8, wherein the disintegrating agent is corn starch, pregelatinized starch, alginic acid, or a combination thereof.
11. The pharmaceutical composition of embodiment 8, wherein the sweetening agent is sucrose, lactose, saccharin or a combination thereof.
12. The pharmaceutical composition of embodiment 8, wherein the flavoring agent is peppermint, oil of wintergreen, cherry, or a combination thereof.
13. The pharmaceutical composition of any of embodiments 1 to 12, further comprising at least one buffering agent.
14. The pharmaceutical composition of any of embodiments 1 to 13, further comprising a fatty oil.
15. The pharmaceutical composition of any of embodiments 1 to 14, wherein the composition is a topical formulation in the form of an ointment, gel, cream, emulsion, oil, foam or spray for dermal application.
16. The pharmaceutical composition of any of embodiments 1 to 15, wherein the compound is administered in the form of a medicament, which comprises also at least one pharmaceutically acceptable diluent, preservative, antioxidant, solubilizer, emulsifier adjuvant or carrier.
17. The pharmaceutical composition of any of embodiments 1 to 16, wherein the medicament is further characterized by at least one of the following features:
   (i) it is adapted for oral, rectal, parenteral, transbuccal, intrapulmonary (e.g. by inhalation) transdermal or ectopic administration;
   (ii) it is in unit dosage form, each unit dosage comprising an amount of said at least one compound at effective dose;
   (iii) it is a prolonged release formulation;
   (iv) it is in a depot form which will release the compound slowly in the body, over a preselected time period;
   (v) it is an ointment, cream, foam or spray intended for ectopic use
   (vi) it comprises also at least one additional therapeutic agent selected from UV protectants, analgesics, minor tranquilizers, and anti- inflammatory drugs.
18. The pharmaceutical composition of any of embodiments 1 to 17, wherein the compound is selected from the group consisting of N-[2-(1H-indol-3-yl)-ethyl]-comanilamide, N-[2-(5-methoxy-indol-3-yl)-ethyl]-comanilamide, and 2-methyl-4-oxo-4H-pyran-3-yl [2-(5-methoxy-1H-indol-3-yl)ethyl]carbamate.
19. The pharmaceutical composition of any of embodiments 1 to 17, wherein the composition comprises the compound of formula I in an amount sufficient to reduce itch or pruritus in a patient in need of such reduction.
20. The pharmaceutical composition of any of embodiments 1 to 17, wherein the compound inhibits Nav1.7 channels.
21. The pharmaceutical composition of any of embodiments 1 to 17, wherein the compound inhibits TRPV channels.
22. A cream formulation for topical application comprising shea butter, coconut oil, and a therapeutically effective amount of a pyrone-indole derivative.
23. The cream formulation of embodiment 22, wherein the pyrone-indole derivative is N-[2-(1H-indol-3-yl)-ethyl]-comanilamide.
24. The cream formulation of embodiment 23, wherein the concentration of the N-[2-(1H-indol-3-yl)-ethyl]-comanilamide is 3%.

### REFERENCES

Akiyama, T., M. I. Carstens and E. Carstens (2010). "Differential itch- and pain-related behavioral responses and micro-opoid modulation in mice." Acta Derm Venereol 90(6): 575-581.
Bautista, D. M., S. R. Wilson and M. A. Hoon (2014). "Why we scratch an itch: the molecules, cells and circuits of itch." Nat Neurosci 17(2): 175-182.
Chuquilin, M., Y. Alghalith and K. H. Fernandez (2016). "Neurocutaneous disease: Cutaneous neuroanatomy and mechanisms of itch and pain." J Am Acad Dermatol 74(2): 197-212.
Furue, M. and T. Kadono (2015). "New therapies for controlling atopic itch." J Dermatol 42(9): 847-850.
Gibson, R. A., J. Robertson, H. Mistry, S. McCallum, D. Fernando, M. Wyres and G. Yosipovitch (2014). "A randomised trial evaluating the effects of the TRPV1 antagonist SB705498 on pruritus induced by histamine, and cowhage challenge in healthy volunteers." PLoS One 9(7): e100610.
Lauffer, F. and J. Ring (2016). "Target-oriented therapy: Emerging drugs for atopic dermatitis." Expert Opin Emerg Drugs 21(1): 81-89.
Lavery, M. J., C. Stull, M. O. Kinney and G. Yosipovitch (2016). "Nocturnal Pruritus: The Battle for a Peaceful Night's Sleep." Int J Mol Sci 17(3).
Lee, J. H., C. K. Park, G. Chen, Q. Han, R. G. Xie, T. Liu, R. R. Ji and S. Y. Lee (2014). "A monoclonal antibody that targets a NaV1.7 channel voltage sensor for pain and itch relief." Cell 157(6): 1393-1404.
Maurer, M., K. Rosen, H. J. Hsieh, S. Saini, C. Grattan, A. Gimenez-Arnau, S. Agarwal, R. Doyle, J. Canvin, A. Kaplan and T. Casale (2013). "Omalizumab for the treatment of chronic idiopathic or spontaneous urticaria." N Engl J Med 368(10): 924-935.
Rice, F. L., P. J. Albrecht, J. P. Wymer, J. A. Black, I. S. Merkies, C. G. Faber and S. G. Waxman (2015). "Sodium channel Nav1.7 in vascular myocytes, endothelium, and innervating axons in human skin." Mol Pain 11: 26.

## Claims

1. A pharmaceutical composition for use in treating a subject suffering from itch or pruritus, said composition comprising an effective amount of at least one compound having the formula:
Ar-B-Ar' (I)
wherein:
-B- represents:
-X-Y-Z-,
wherein:
X represents -(CH₂)ₙ (wherein n is 0-6);
Y represents oxygen, sulphur, >NH or is absent;
Z represents >C=O >O or >COO or is absent; and wherein at least one of X,
Y and Z must be present;
Ar represents an indole nucleus ring system: 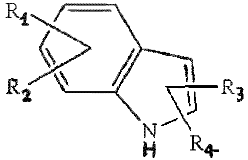
Ar' represents an alpha-, beta- or gamma-pyrone nucleus ring system: 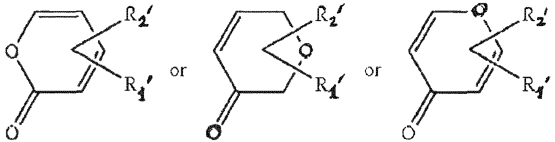
wherein each of R₁₋₄ substitutes the ring system Ar at any available position (including the N-position) and each of R_{1'}-R_{2'} substitutes the ring system Ar' at any available position and wherein each of R₁₋₄ and R_{1'-2'} independently represents hydrogen, oxygen, halo, halo-C₁₋₅ alkyl, aryl, acyl, a C₅₋₇ heterocyclic group containing 1-3 hetero atoms independently selected from nitrogen, oxygen or sulphur; a C₆₋₈ heteroaryl group containing 1-3 hetero atoms independently selected from nitrogen, oxygen or sulphur, C₁₋₅ alkyl, C₂₋₅ alkenyl, C₂₋₅ alkynyl, aryl- C₁₋₅ alkyl, aryl- C₂₋₅ alkenyl, aryl-C₂₋₅ alkynyl, hydroxy-C₁₋₅ alkyl, nitro, amino, cyano, cyanamide, guanidino, amidino, acylamido, C₁₋₅ alkylamine, C₁₋₅ alkylamido, hydroxy, thiol, acyloxy, azido, C₁₋₅ alkoxy, carboxy, carbonylamido or styryl; wherein said arylalkyl, arylalkenyl, aralalkynyl, or styryl group optionally can be ring-substituted by one to four substituents independently selected from the group consisting of hydrogen, halo, halo-C₁₋₅ alkyl, aryl, a C₅₋₇ heterocyclic group containing 1-3 hetero atoms independently selected from nitrogen, oxygen and sulphur; a heteroaryl group containing 1-3 hetero atoms independently selected from nitrogen, oxygen and sulphur; C₁₋₅ alkyl, C₂₋₅ alkenyl, C₂₋₅ alkynyl, aryl-C-₁₋₅ alkyl, aryl-C₂₋₅ alkenyl, aryl-C₂₋₅alkynyl, hydroxy-C₁₋₅ alkyl, nitro, amino, cyano, cyanamido, guanidino, amidino, acylamido, hydroxy, thiol, acyloxy, azido, alkoxy, carboxy, carbonylamido, S-alkyl or alkylthiol;
and either of R₃ or R₄ further can include or represent a bond to B;
wherein Ar is bonded to B at position 3 on the five-membered ring portion of the Ar ring and Ar' can be bonded to B at any carbon on the Ar' ring not substituted by R_{1'} and R_{2'};
or a salt, stereoisomer, or racemic mixture thereof, and
at least one pharmaceutically acceptable diluent, preservative, antioxidant, solubilizer, emulsifier, gelling agent, adjuvant or carrier.

2. The pharmaceutical composition for use of claim 1, wherein X is -(CH₂)ₙ, wherein n is 0-6, Y is >NH or >O and Z is >CO.

3. The pharmaceutical composition for use of claim 2, wherein Ar' is an alpha-pyrone ring system.

4. The pharmaceutical composition for use of claim 2, wherein Ar' is a beta-pyrone ring system.

5. The pharmaceutical composition for use of claim 2, wherein Ar' is a gamma-pyrone ring system.

6. The pharmaceutical composition for use of claim 1, wherein X is -(CH₂)ₙ, Y is >NH or >O, and Z is >CO, Ar is an indole ring; R₃ is a bond to X on position 3 of the indole ring; R₁ is hydrogen or a methoxy group on position 5 of the indole ring, and each of R₂ and R₄ is hydrogen; Ar' is a gamma-pyrone ring bonded to Z at position 2 of the pyrone ring; R_{1'} is hydrogen or a hydroxy group on position 5 of the pyrone ring; and R_{2'} is hydrogen or a carboxy group on position 6 of the gamma-pyrone ring; or a pharmaceutically acceptable salt, stereoisomer, or racemic mixture thereof.

7. The pharmaceutical composition for use of claim 1, wherein X is -(CH₂)ₙ, Y is >NH or >O and Z is >COO; Ar is an indole ring; R₃ is a bond to X on position 3 of the indole ring; R₁ is a methoxy group at position 5 of the indole ring and each of R₂ and R₄ is hydrogen; Ar' is an gamma-pyrone ring substituted by Z at position 4 of the pyrone ring; and R₁, and R₂ are each a methyl or hydrogen; or a pharmaceutically acceptable salt, stereoisomer, or racemic mixture thereof.

8. The pharmaceutical composition for use of claim 1, wherein the compound of formula (I) is selected from the group consisting of N-[2-(1H-indol-3-yl)-ethyl]-comanilamide, N-[2-(5-methoxy-indol-3-yl)-ethyl]-comanilamide, and 2-methyl-4-oxo-4H-pyran-3-yl [2-(5-methoxy-1H-indol-3-yl)ethyl]carbamate.

9. The pharmaceutical composition for use of claims 1 to 8, wherein the composition is further **characterized by** at least one of the following features:
(i) it is adapted for oral, rectal, parenteral, transbuccal, intrapulmonary (e.g. by inhalation) transdermal or topical administration;
(ii) it is in unit dosage form, each unit dosage comprising an effective amount of said at least one compound;
(iii) it is a prolonged release formulation;
(iv) it is in a depot form which will release the compound slowly in the body, over a preselected time period;
(v) it is an ointment, cream, gel, emulsion, oil, foam, solution or aerosol spray suitable for topical use;
(vi) it further comprises at least one additional therapeutic agent selected from a UV protectant, an analgesic, a tranquilizer, a vasoconstrictor, a vasodilator, and an anti-inflammatory agent.

10. The pharmaceutical composition for use of claim 9, wherein the composition is a solid oral dosage form selected from capsules, tablets, pills, powders or granules, wherein preferably the compound of formula I is admixed with at least one inert pharmaceutically acceptable carrier selected from sucrose, lactose or starch.

11. The pharmaceutical composition for use of claim 10, wherein the composition further comprises one or more lubricating agents, and preferably comprises magnesium stearate.

12. The pharmaceutical composition for use of claims 10 or 11, wherein the composition further comprises one or more adjuvants selected from:
- binders, preferably gum tragacanth, acacia, corn starch or gelatin,
- excipients, preferably microcrystalline cellulose;
- disintegrating agents, preferably corn starch, pregelatinized starch or alginic acid;
- sweetening agents, preferably sucrose, lactose or saccharin;
- flavoring agents, preferably peppermint, oil of wintergreen or cherry.

13. The pharmaceutical composition for use of claim 9, wherein the composition is a topical formulation in the form of an ointment, a gel, a cream, an emulsion, an oil, a foam or a spray for dermal application.

14. The pharmaceutical composition for use of claim 1, wherein the composition is a cream formulation comprising shea butter, coconut oil and a therapeutically effective amount of a pyrone-indole derivative of formula (I).

15. The pharmaceutical composition for use of claim 14, wherein the pyrone-indole derivative of formula (I) is N-[2-(1H-indol-3-yl)-ethyl]-comanilamide, preferably at a concentration from 1 to 10% or from 2 to 8% or from 3 to 5% or of 3%.

## Patentansprüche

1. Pharmazeutische Zusammensetzung zur Verwendung bei der Behandlung eines Individuums, das an Juckreiz oder Pruritus leidet, wobei die Zusammensetzung eine wirksame Menge mindestens einer Verbindung mit der Formel:
Ar-B-Ar' (I)
umfasst,
wobei:
-B- darstellt:
-X-Y-Z-,
wobei:
X -(CH₂)ₙ darstellt (wobei n 0-6 ist);
Y Sauerstoff, Schwefel, >NH darstellt oder abwesend ist;
Z >C=O, >O oder >COO darstellt oder abwesend ist; und wobei mindestens eines von X, Y und Z vorhanden sein muss;
Ar ein Indol-Kern-Ringsystem darstellt: 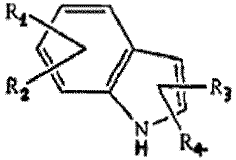
Ar' ein Alpha-, Beta- oder Gamma-Pyron-Kern-Ringsystem darstellt: 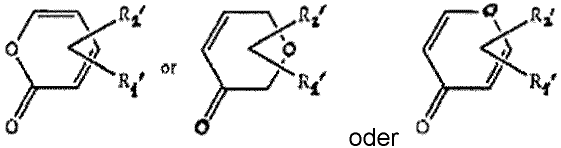
wobei jedes aus R₁₋₄ das Ringsystem Ar an einer beliebigen verfügbaren Position (einschließlich der N-Position) substituiert und jedes aus R_{1'}-R_{2'} das Ringsystem Ar' an einer beliebigen verfügbaren Position substituiert und wobei jedes aus R₁₋₄ und R_{1'-2'} unabhängig Wasserstoff, Sauerstoff, Halogen, Halogen-Ci-s-Alkyl, Aryl, Acyl, eine heterocyclische C₅₋₇-Gruppe, die 1-3 Heteroatome enthält, die unabhängig ausgewählt sind aus Stickstoff, Sauerstoff oder Schwefel, eine C₆₋₈-Heteroarylgruppe, die 1-3 Heteroatome enthält, die unabhängig ausgewählt sind aus Stickstoff, Sauerstoff oder Schwefel, C₁₋₅-Alkyl, C₂₋₅-Alkenyl, C₂₋₅-Alkinyl, Aryl-C₁₋₅-Alkyl, Aryl-C₂₋₅-alkenyl, Aryl-C₂₋₅-Alkinyl, Hydroxy-Ci-s-Alkyl, Nitro, Amino, Cyano, Cyanamid, Guanidino, Amidino, Acylamido, C₁₋₅-Alkylamin, C₁₋₅-Alkylamido, Hydroxy, Thiol, Acyloxy, Azido, C₁₋₅-Alkoxy, Carboxy, Carbonylamido oder Styryl; wobei die Arylalkyl-, Arylalkenyl-, Arylalkinyl- oder Styrylgruppe gegebenenfalls Ring-substituiert sein kann durch einen bis vier Substituenten, die unabhängig ausgewählt sind aus der Gruppe bestehend aus Wasserstoff, Halogen, Halogen-C₁₋₅-Alkyl, Aryl, einer heterocyclischen C₅₋₇-Gruppe, die 1-3 Heteroatome enthält, die unabhängig ausgewählt sind aus Stickstoff, Sauerstoff und Schwefel, einer Heteroarylgruppe, die 1-3 Heteroatome enthält, die unabhängig ausgewählt sind aus Stickstoff, Sauerstoff und Schwefel; C₁₋₅-Alkyl, C₂₋₅-Alkenyl, C₂₋₅-Alkinyl, Aryl-C₁₋₅-Alkyl, Aryl-C₂₋₅-Alkenyl, Aryl-C₂₋₅-Alkinyl, Hydroxy-C₁₋₅-Alkyl, Nitro, Amino, Cyano, Cyanamido, Guanidino, Amidino, Acylamido, Hydroxy, Thiol, Acyloxy, Azido, Alkoxy, Carboxy, Carbonylamido, S-Alkyl oder Alkylthiol;
und jedes aus R₃ oder R₄ weiterhin eine Bindung zu B einschließen oder darstellen kann;
wobei Ar an B an der Position 3 auf dem fünfgliedrigen Ringteil des Ar-Rings gebunden ist und Ar' an B an einem beliebigen Kohlenstoff auf dem Ar'-Ring, der nicht durch R_{1'} und R_{2'} substituiert ist, gebunden sein kann;
oder ein Salz, Stereoisomer oder racemisches Gemisch davon, und
mindestens ein(en) pharmazeutisch annehmbares/n Verdünnungsmittel, Konservierungsmittel, Antioxidationsmittel, Lösungsvermittler, Emulgator, Geliermittel, Adjuvans oder Träger.

2. Pharmazeutische Zusammensetzung zur Verwendung nach Anspruch 1, wobei X -(CH₂)ₙ ist, wobei n 0-6 ist, Y >NH oder >O ist und Z >CO ist.

3. Pharmazeutische Zusammensetzung zur Verwendung nach Anspruch 2, wobei Ar' ein alpha-Pyron-Ringsystem ist.

4. Pharmazeutische Zusammensetzung zur Verwendung nach Anspruch 2, wobei Ar' ein Beta-Pyron-Ringsystem ist.

5. Pharmazeutische Zusammensetzung zur Verwendung nach Anspruch 2, wobei Ar' ein Gamma-Pyron-Ringsystem ist.

6. Pharmazeutische Zusammensetzung zur Verwendung nach Anspruch 1, wobei X -(CH₂)ₙ ist, Y >NH oder>O ist und Z>CO ist, Ar ein Indolring ist; R₃ eine Bindung zu X an Position 3 des Indolrings ist; R₁ Wasserstoff oder eine Methoxygruppe an Position 5 des Indolrings ist und jedes aus R₂ und R₄ Wasserstoff ist; Ar' ein Gamma-Pyronring ist, der an Z an Position 2 des Pyronrings gebunden ist; R_{1'} Wasserstoff oder eine Hydroxygruppe an Position 5 des Pyronrings ist und R_{2'} Wasserstoff oder eine Carboxygruppe an Position 6 des Gamma-Pyronrings ist; oder ein pharmazeutisch annehmbares Salz, Stereoisomer oder racemisches Gemisch davon.

7. Pharmazeutische Zusammensetzung zur Verwendung nach Anspruch 1, wobei X -(CH₂)ₙ ist, Y >NH oder >O ist und Z>COO ist; Ar ein Indolring ist; R₃ eine Bindung zu X an Position 3 des Indolrings ist; R₁ eine Methoxygruppe an Position 5 des Indolrings ist und jedes aus R₂ und R₄ Wasserstoff ist; Ar' ein Gamma-Pyronring ist, der durch Z an Position 4 des Pyronrings substituiert ist und R₁ und R₂ jeweils ein Methyl oder Wasserstoff sind; oder ein pharmazeutisch annehmbares Salz, Stereoisomer oder racemisches Gemisch davon.

8. Pharmazeutische Zusammensetzung zur Verwendung nach Anspruch 1, wobei die Verbindung der Formel (I) ausgewählt ist aus der Gruppe bestehend aus N-[2-(1H-Indol-3-yl)-ethyl]-comanilamid, N-[2-(5-Methoxy-indol-3-yl)-ethyl]-comanilamid und 2-Methyl-4-oxo-4H-pyran-3-yl-[2-(5-methoxy-1H-indol-3-yl)-ethyl]-carbamat.

9. Pharmazeutische Zusammensetzung zur Verwendung nach den Ansprüchen 1 bis 8, wobei die Zusammensetzung weiterhin durch mindestens eines der folgenden Merkmale gekennzeichnet ist:
(i) sie ist für eine orale, rektale, parenterale, transbukkale, intrapulmonale (z.B. durch Inhalation), transdermale oder topische Verabreichung geeignet;
(ii) sie liegt in Dosierungseinheiten-Form vor, wobei jede Dosierungseinheit eine wirksame Menge der mindestens einen Verbindung umfasst;
(iii) sie ist eine Formulierung mit verlängerter Freisetzung;
(iv) sie liegt in einer Depot-Form vor, die die Verbindung langsam in den Körper über einen vorgewählten Zeitraum freisetzen wird;
(v) sie ist ein(e) Salbe, Creme, Gel, Emulsion, Öl, Schaum, Lösung oder Aerosolspray, geeignet für die topische Anwendung;
(vi) sie umfasst weiterhin mindestens ein zusätzliches therapeutisches Mittel, ausgewählt aus einem UV-Schutzmittel, einem Analgetikum, einem Beruhigungsmittel, einem Vasokonstriktor, einem Vasodilatator und einem entzündungshemmenden Mittel.

10. Pharmazeutische Zusammensetzung zur Verwendung nach Anspruch 9, wobei die Zusammensetzung eine feste orale Darreichungsform ist, ausgewählt aus Kapseln, Tabletten, Pillen, Pulvern oder Granulaten, wobei bevorzugt die Verbindung der Formel I mit mindestens einem inerten pharmazeutisch annehmbaren Träger, ausgewählt aus Saccharose, Lactose oder Stärke, gemischt ist.

11. Pharmazeutische Zusammensetzung zur Verwendung nach Anspruch 10, wobei die Zusammensetzung weiterhin ein oder mehrere Gleitmittel umfasst, und bevorzugt Magnesiumstearat umfasst.

12. Pharmazeutische Zusammensetzung zur Verwendung nach den Ansprüchen 10 oder 11, wobei die Zusammensetzung weiterhin ein oder mehrere Adjuvantien umfasst, ausgewählt aus:
- Bindemitteln, bevorzugt Tragant, Akazie, Maisstärke oder Gelatine,
- Hilfsstoffen, bevorzugt mikrokristalliner Cellulose;
- Sprengmitteln, bevorzugt Maisstärke, vorgelatinierte Stärke oder Alginsäure;
- Süßungsmitteln, bevorzugt Saccharose, Lactose oder Saccharin;
- Aromastoffen, bevorzugt Pfefferminz, Öl von Wintergrün oder Kirsche.

13. Pharmazeutische Zusammensetzung zur Verwendung nach Anspruch 9, wobei die Zusammensetzung eine topische Formulierung in Form einer Salbe, eines Gels, einer Creme, einer Emulsion, eines Öls, eines Schaums oder eines Sprays zur dermalen Anwendung ist.

14. Pharmazeutische Zusammensetzung zur Verwendung nach Anspruch 1, wobei die Zusammensetzung eine Cremeformulierung ist, die Sheabutter, Kokosnussöl und eine therapeutisch wirksame Menge eines Pyron-Indol-Derivats der Formel (I) umfasst.

15. Pharmazeutische Zusammensetzung zur Verwendung nach Anspruch 14, wobei das Pyron-Indol-Derivat der Formel (I) N-[2-(1H-Indol-3-yl)-ethyl]-comanilamid ist, bevorzugt bei einer Konzentration von 1 bis 10% oder von 2 bis 8% oder von 3 bis 5% oder 3%.

## Revendications

1. Composition pharmaceutique pour l'utilisation dans le traitement d'un sujet soufrant de démangeaison ou de prurit, ladite composition comprenant une quantité efficace d'au moins un composé ayant la formule :
Ar-B-Ar' (I)
dans laquelle :
-B- représente :
-X-Y-Z-,
dans laquelle :
X représente -(CH₂)ₙ (où n a la valeur de 0 à 6) ;
Y représente un atome d'oxygène, de soufre, > NH ou est absent ;
Z représente > C=O, > O ou > COO ou est absent ; et dans laquelle au moins l'un de X, Y et Z doit être présent ;
Ar représente un système cyclique à noyau indole : 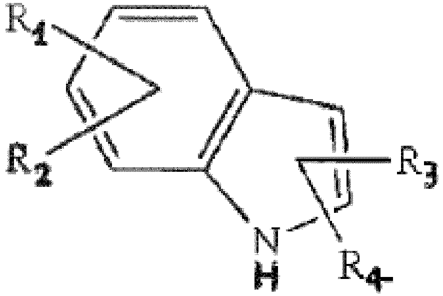
Ar' représente un système cyclique à noyau alpha-, bêta- ou gamma-pyrone : 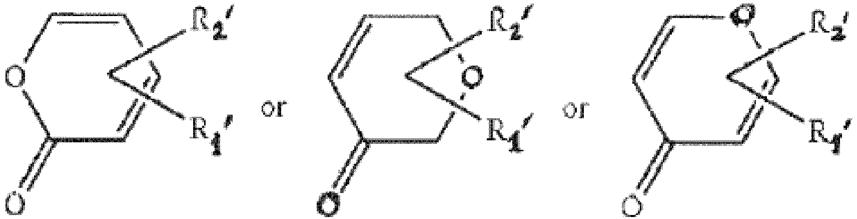
chacun de R₁₋₄ substitue le système cyclique Ar à n'importe quelle position disponible (y compris la position N) et chacun de R_{1'}-R_{2'} substitue le système cyclique Ar' à n'importe quelle position disponible et chacun de R₁₋₄ et R_{1'-2'} représente indépendamment un atome d'hydrogène, d'oxygène, un groupe halogéno, halogéno-(alkyle en C₁ à C₅), aryle, acyle, un groupe hétérocyclique en C₅ à C₇ contenant de 1 à 3 hétéroatomes indépendamment sélectionnés parmi l'atome d'azote, d'oxygène ou de soufre ; un groupe hétéroaryle en C₆ à C₈ contenant de 1 à 3 hétéroatomes indépendamment sélectionnés parmi l'atome d'azote, d'oxygène ou de soufre, un groupe alkyle en C₁ à C₅, alcényle en C₂ à C₅, alcynyle en C₂ à C₅, aryl-(alkyle en C₁ à C₅), aryl-(alcényle en C₂ à C₅), aryl-(alcynyle en C₂ à C₅), hydroxy-(alkyle en C₁ à C₅), nitro, amino, cyano, cyanamide, guanidino, amidino, acylamido, alkylamine en C₁ à C₅, alkylamido en C₁ à C₅, hydroxy, thiol, acyloxy, azido, alcoxy en C₁ à C₅, carboxyle, carbonylamido ou styryle ; ledit groupe arylalkyle, arylalcényle, aralalcynyle, ou styryle éventuellement pouvant être substitué sur le cycle par un à quatre substituants indépendamment sélectionnés parmi le groupe constitué du groupe hydrogéno, halogéno, halogéno-(alkyle en C₁ à C₅), aryle, un groupe hétérocyclique en C₅ à C₇ contenant de 1 à 3 hétéroatomes indépendamment sélectionnés parmi l'atome d'azote, d'oxygène et de soufre ; un groupe hétéroaryle contenant de 1 à 3 hétéroatomes indépendamment sélectionnés parmi l'atome d'azote, d'oxygène et de soufre ; un groupe alkyle en C₁ à C₅, alcényle en C₂ à C₅, alcynyle en C₂ à C₅, aryl-(alkyle en C₁ à C₅), aryl-(alcényle en C₂ à C₅), aryl-(alcynyle en C₂ à C₅), hydroxy-(alkyle en C₁ à C₅), nitro, amino, cyano, cyanamido, guanidino, amidino, acylamido, hydroxy, thiol, acyloxy, azido, alcoxy, carboxyle, carbonylamido, S-alkyle ou alkylthiol ;
et l'un ou l'autre de R₃ ou R₄ en outre peut comprendre ou représenter une liaison à B ;
Ar étant lié à B à la position 3 sur la portion cyclique à cinq chaînons du cycle Ar et Ar' peut être lié à B à n'importe quel atome de carbone sur le cycle Ar' non substitué par R_{1'} et R_{2'} ;
ou un sel, un stéréoisomère, ou un mélange racémique de celui-ci, et au moins un diluant, conservateur, antioxydant, agent de solubilisation, émulsifiant, agent gélifiant, adjuvant ou support pharmaceutiquement acceptable.

2. Composition pharmaceutique pour l'utilisation selon la revendication 1, X étant -(CH₂)ₙ, n ayant la valeur de 0 à 6, Y étant > NH ou > O et Z étant > CO.

3. Composition pharmaceutique pour l'utilisation selon la revendication 2, Ar' étant un système cyclique alpha-pyrone.

4. Composition pharmaceutique pour l'utilisation selon la revendication 2, Ar' étant un système cyclique bêta-pyrone.

5. Composition pharmaceutique pour l'utilisation selon la revendication 2, Ar' étant un système cyclique gamma-pyrone.

6. Composition pharmaceutique pour l'utilisation selon la revendication 1, X étant -(CH₂)ₙ, Y étant > NH ou > O, et Z étant > CO, Ar étant un cycle indole ; R₃ étant une liaison à X sur la position 3 du cycle indole ; R₁ étant un groupe hydrogéno ou méthoxy sur la position 5 du cycle indole, et chacun de R₂ et R₄ étant un groupe hydrogéno ; Ar' étant un cycle gamma-pyrone lié à Z à la position 2 du cycle pyrone ; R_{1'} étant un groupe hydrogéno ou hydroxyle sur la position 5 du cycle pyrone ; et R_{2'} étant un groupe hydrogéno ou carboxyle sur la position 6 du cycle gamma-pyrone ; ou un sel, stéréoisomère, ou mélange racémique de celui-ci pharmaceutiquement acceptable.

7. Composition pharmaceutique pour l'utilisation selon la revendication 1, X étant -(CH₂)ₙ, Y étant > NH ou > O et Z étant > COO ; Ar étant un cycle indole ; R₃ étant une liaison à X sur la position 3 du cycle indole ; R₁ étant un groupe méthoxy à la position 5 du cycle indole et chacun de R₂ et R₄ étant un groupe hydrogéno ; Ar' étant un cycle gamma-pyrone substitué par Z à la position 4 du cycle pyrone ; et R₁, et R₂ étant chacun un groupe méthyle ou hydrogéno ; ou un sel, stéréoisomère, ou mélange racémique de celui-ci pharmaceutiquement acceptable.

8. Composition pharmaceutique pour l'utilisation selon la revendication 1, le composé de formule (I) étant sélectionné dans le groupe constitué du N-[2-(1H-indol-3-yl)-éthyl]-comanilamide, du N-[2-(5-méthoxy-indol-3-yl)-éthyl]-comanilamide, et du [2-(5-méthoxy-1H-indol-3-yl)éthyl]carbamate de 2-méthyl-4-oxo-4H-pyran-3-yle.

9. Composition pharmaceutique pour l'utilisation selon les revendications 1 à 8, la composition étant en outre **caractérisée par** au moins l'une des caractéristiques suivantes :
(i) elle est adaptée à l'administration par voie orale, rectale, parentérale, transbuccale, intrapulmonaire (par exemple par inhalation) transdermique ou topique ;
(ii) elle se présente sous une forme posologique unitaire, chaque dose unitaire comprenant une quantité efficace dudit au moins un composé ;
(iii) elle est une formulation à libération prolongée ;
(iv) elle se présente sous une forme de dépôt qui libérera le composé lentement dans le corps, sur une période de temps présélectionnée ;
(v) elle est un onguent, une crème, un gel, une émulsion, une huile, une mousse, une solution ou une pulvérisation par aérosol convenant à l'utilisation par voie topique ;
(vi) elle comprend en outre au moins un agent thérapeutique additionnel sélectionné parmi un agent protecteur vis-à-vis du rayonnement UV, un analgésique, un agent tranquillisant, un vasoconstricteur, un vasodilatateur, et un agent anti-inflammatoire.

10. Composition pharmaceutique pour l'utilisation selon la revendication 9, la composition étant une forme posologique solide par voie orale sélectionnée parmi les capsules, les comprimés, les pilules, les poudres ou les granules, préférablement le composé de formule I est mélangé par admixtion avec au moins un support inerte pharmaceutiquement acceptable sélectionné parmi le saccharose, le lactose ou l'amidon.

11. Composition pharmaceutique pour l'utilisation selon la revendication 10, la composition comprenant en outre un ou plusieurs agents lubrifiants, et comprenant préférablement du stéarate de magnésium.

12. Composition pharmaceutique pour l'utilisation selon les revendications 10 ou 11, la composition comprenant en outre un ou plusieurs adjuvants sélectionnés parmi :
- les liants, préférablement la gomme adragante, l'acacia, l'amidon de maïs ou la gélatine,
- les excipients, préférablement la cellulose microcristalline ;
- les agents délitants, préférablement l'amidon de maïs, l'amidon prégélatinisé ou l'acide alginique ;
- les agents édulcorants, préférablement le saccharose, le lactose ou la saccharine ;
- les agents aromatisants, préférablement la menthe poivrée, l'huile de gaulthérie couchée ou de cerise.

13. Composition pharmaceutique pour l'utilisation selon la revendication 9, la composition étant une formulation topique se présentant sous la forme d'un onguent, d'un gel, d'une crème, d'une émulsion, d'une huile, d'une mousse ou d'une pulvérisation pour l'application dermique.

14. Composition pharmaceutique pour l'utilisation selon la revendication 1, la composition étant une formulation de crème comprenant du beurre de karité, de l'huile de noix de coco et une quantité thérapeutiquement efficace d'un dérivé pyrone-indole de formule (I).

15. Composition pharmaceutique pour l'utilisation selon la revendication 14, le dérivé pyrone-indole de formule (I) étant le N-[2-(1H-indol-3-yl)-éthyl]-comanilamide, préférablement sous une concentration de 1 à 10 % ou de 2 à 8 % ou de 3 à 5 % ou de 3 %.
